(19) **Europäisches Patentamt**

**European Patent Office**

**Office européen des brevets**

(11) Publication number: **0 170 927 B1**

(12) # EUROPEAN PATENT SPECIFICATION

(45) Date of publication of patent specification: **20.02.91**  (51) Int. Cl.⁵: **A61K 7/08**

(21) Application number: **85108640.5**

(22) Date of filing: **11.07.85**

(54) Detergent compositions.

(30) Priority: **02.08.84 GB 8419737**

(43) Date of publication of application:
**12.02.86 Bulletin 86/07**

(45) Publication of the grant of the patent:
**20.02.91 Bulletin 91/08**

(84) Designated Contracting States:
**AT BE CH DE FR GB IT LI LU NL SE**

(56) References cited:
**EP-A- 0 074 264**     **FR-A- 1 414 966**
**FR-A- 2 350 835**     **GB-A- 2 107 586**
**US-A- 3 998 761**     **US-A- 4 292 212**

(73) Proprietor: **BEECHAM GROUP PLC**
**Beecham House Great West Road**
**Brentford Middlesex TW8 9BD(GB)**

(72) Inventor: **Wilkins, Anne Lilian**
**77 Strawberry Vale Twickenham**
**Middlesex(GB)**
Inventor: **Stothard, Heather Elizabeth**
**325 Hersham Road Hersham**
**Walton-on-Thames Surrey(GB)**
Inventor: **Clarkson, Quinten Robert Mark**
**55 Byfleet Road New Haw**
**Weybridge Surrey(GB)**

(74) Representative: **Russell, Brian John et al**
**Beecham Pharmaceuticals Great Burgh Yew**
**Tree Bottom Road**
**Epsom Surrey KT18 5XQ(GB)**

Rank Xerox (UK) Business Services

EP 0 170 927 B1

**Description**

The present invention relates to detergent compositions and in particular to a shampoo composition in rigid gel form, comprising from 5 to 25% w/w of anionic detergent (based upon 100 % active material) and from 0.5 to 7% by weight of the total composition of a thickening agent which has secondary detergent properties.

A composition of this type is described in GB-A-2 107 586 having two contiguous gel bodies, one containing detergent, the other containing an insoluble anti-dandruff agent, zinc pyridinethione (herein referred to as ZPT). The high viscosity of the gel body enables the insoluble anti-dandruff agent to be suspended therein without the need for a suspending agent, and also ensures that the two gel bodies are self supporting and non-merging.

A disadvantage of gel compositions of this type, which depend for their rigidity on the fact that the anionic detergent is in its "M-phase", is that they tend to show large variations in viscosity during manufacture. The observed variations are known to be caused by small variations in the concentrations of inorganic ions in the composition and hence variations in the concentrations of those constituents which are sources of such ions. A major source of inorganic ions in shampoo compositions is the detergent. The concentrations of ions tend to vary from batch to batch of detergent making it difficult to control these concentrations during manufacture. It would be most advantageous therefore if a less sensitive rigid gel composition could be identified.

It has now suprisingly been found that the incorporation of certain humectant polymers into the gel composition provides a composition with a viscosity which is much less sensitive to variations in the levels of constituent inorganic ions.

The term 'humectant polymer' is used herein to mean a polymer having an affinity for water and the ability to regulate the free water content of the medium in which it is incorporated.

Accordingly, the rigid gel shampoo compositions of the present invention are characterized by the addition of from 0.1 to 5% by weight of a stabilising humectant polymer selected from non-ionic guar gums, hydroxy $C_{1-6}$ alkyl ethers and hydroxy $C_{1-6}$ alkyl esters of cellulose.

Suitable humectant polymers are known per se , for example for their thickening properties. FR-A-2 350 835 describes the optional use of a hydroxy alkyl cellulose for thickening a non-rigid shampoo product. However, the use of such polymers for stabilizing an M-phase product has not previously been proposed.

The composition of the invention may also contain an anti-dandruff agent.

Preferred humectant polymers include hydroxy propyl guar gum and hydroxy ethyl cellulose.

A particularly preferred form of hydroxy propyl guar gum is that marketed under the Trade Name of Jaguar HP 60, and a preferred form of hydroxy ethyl cellulose is that marketed under the Trade Name of Natrosol 250 HHR.

The composition of the invention preferably contains about 0.5% w/w of the humectant polymer.

The anti-dandruff agent may be soluble or insoluble in the composition of the invention although preferred anti-dandruff agents are insoluble. A particularly preferred anti-dandruff agent is ZPT and this is suitably present in the composition in an amount of 0.1% to 2.5% by weight of the total composition.

Suitably the composition is contained in a bottle, pump-pack, tub or tube, preferably in a collapsible tube.

In one preferred aspect, the composition of the invention comprises a first gel body containing the detergent and the humectant polymer and a second gel body contiguous with the first gel body, containing the anti-dandruff agent and, optionally, the same humectant polymer.

Suitably the first gel body is transparent or translucent and the second gel body is substantially opaque.

In the aforementioned preferred aspect of the invention the composition is suitably contained in a tube or other container which is preferably collapsible, so that when the composition is discharged from the container the two gel bodies remain in contact with each other and form a self supporting gel structure.

The consistencies of the two gel bodies should be sufficiently firm to hold the bodies together in position, and yet be easily extrudable through, for example, the nozzle of a tube. Preferably, the two gel bodies have substantially identical rheological properties.

The weight ratios of the two bodies can vary within wide limits. For example, the weight ratio of the first gel body to the second gel body may be from 1:9 to 9:1. A particularly suitable ratio is 1:1. The distribution of the two gel bodies can also vary widely in order to give different visual patterns. For example, one gel body may be completely contained within the other gel body, or one gel may be partly contained in the other gel body to form a stripe or stripes.

The desired rheological properties of the gel bodies may be achieved by incorporating suitable thickening materials into the bodies.

It has been found desirable to use as a thickening agent a material which has secondary detergent and/or foam stabilising properties. One or more such foam stabiliser/thickener materials may be present in each gel body, and preferred materials are lauric isopropanolamide and coconut monoethanolamide. The amount of such a foam stabiliser/thickener is from 0.5 to 7.0% by weight of the total composition.

Preferably each gel body contains one or more agents designed to improve the cosmetic condition of the hair after shampooing, by enhancing feel and gloss and reducing electrostatic flyaway. Such agents may comprise wholly or in part the detergent materials described above as providing a foam stabilising or thickening effect, or may comprise other agents with surface active properties or certain polymeric materials. Suitable surface active materials are certain salts of ethoxylated fatty alcohol phosphate esters, particularly an oleyl alcohol derived material marketed under the trade name Briphos 03D. Suitable polymeric materials are cationic cellulosic resins marketed under the trade name Polymer JR 125,400 or 30M or quarternary of a vinyl pyrrolidone copolymer marketed under the trade name Gafquat. 755N. A particularly effective material is a cationic guar gum sold under the trade name Jaguar C-I3-S by Meyhall Chemicals Ltd. This may be present in either or both gel bodies in an amount from 0.1 to 5.0% by weight of the total composition.

The compositions according to the invention may include any conventional anionic detergent well known in the art for example alkali metal, ammonium or hydroxyalkylamine salts of alkyl sulphates or alkyl ether sulphates, alkyl benzene sulphonates, alkyl sulphones, α-alkenyl sulphonates, polyoxyethylenealkyl sulphonates and polyoxyethylenealkylphenylsulphonates. A preferred detergent comprises sodium lauryl ether sulphate.

The composition of the invention may also contain various conventional accessory ingredients such as perfumes, colouring materials and preservatives.

The present invention also provides a process for the preparation of a detergent composition, which comprises:

a. admixing the humectant polymer and the detergent in water, suitably at an elevated temperature preferably 70 to 75°C;

b. and optionally adding the anti-dandruff agent.

Additional ingredients such as thickening agents, foam stabilisers, conditioning agents, opacifying agents, dyes, perfumes and preservatives may be added at any convenient time during the process. Suitably the dyes, perfumes and preservatives are added after the addition of the anti-dandruff agent preferably at ambient or slightly above ambient temperature.

In a further aspect the present invention also provides a process for the preparation of the aforementioned preferred form of the composition, wherein the first gel body is prepared according to step a) and may optionally include additional water; the second gel body is prepared according to steps a) and b).

In the preferred form of the present invention the compositions may be made by charging a suitable container with the two gel bodies, using the method and apparatus described in British Patent Specification No. 962,757.

The present invention will now be illustrated by the following Examples.

## EXAMPLE 1

| Ingredient | % w/w | |
|---|---|---|
| | First Gel Body | Second Gel Gel |
| SLES(28%) | 70.0 | 70.0 |
| Lauric isopropanolamide | 3.0 | 2.88 |
| Coconut monoethanolamide | 3.0 | 2.88 |
| Cationic guar gum | 0.5 | 0.5 |
| Hydroxy propyl guar gum | 0.5 | 0.5 |
| ZPT | – | 2.0 |
| Perfume, dyes, preservatives, etc | q.s. | q.s. |
| Water to | 100.0% | 100.0% |

SLES (28%) refers to 28% w/w aqueous solution of sodium lauryl ether sulphate.
The weight ratio of the two gel bodies is 1:1.

## EXAMPLE 2

| Ingredient | % w/w Gel Body |
|---|---|
| SLES (28%) | 70.0 |
| Lauric isopropanolamide | 3.0 |
| Coconut monoethanolamide | 3.0 |
| Cationic guar gum | 0.5 |
| Hydroxyethyl cellulose | 0.5 |
| Perfume, dyes, preservatives etc | q.s. |
| Water to | 100.0% |

## EXAMPLE 3

| Ingredient | % w/w Gel Body |
|---|---|
| Triethanolamine lauryl sulphate | 70.0 |
| Linoleic diethanolamide | 6.0 |
| Quaternary derivative of a vinyl pyrrolidone copolymer | 0.25 |
| Hydroxy propyl guar gum | 0.50 |
| Perfume, dyes, preservatives, etc. | q.s. |
| Water to | 100.0% |

EPLE 2 v/w Ingredient Gel Body SLES (28) 70.0 -uric isopropanolide 3.0 conut monoethanolide 3.0 Cationii: guar gum 0.5 Bydroxyethyl cellulose 0.5 Perfume, dyes, preservatives etc q.s. Water to l00.0 EP 3 w/w Ingredient Gel Body Triethanoline lauryl sulphate 70.0 Linoleic diethanolide 6.0 Quaternary derivative of a vinyl pyrrolidone copolrner 0.25 - Hydroxy propyl guar gum 0.50 Perfume, dyes, Preservatives, etc. q.s. Water to l00.0

## EXAMPLE 4

| Ingredient | % w/w<br>Gel Body |
|---|---|
| SLES (28%) | 70.0 |
| Lauric isopropanolamide | 3.0 |
| Coconut monoethanolamide | 3.0 |
| Cationic guar gum | 0.5 |
| Hydroxyl propyl guar gum | 0.5 |
| Piroctone Olamine | 0.5 |
| Perfume, dyes, preservatives, etc. | q.s. |
| Water to | 100.0% |

## EXAMPLE 5

| Ingredient | % w/w | |
|---|---|---|
| | First Gel Body | Second Gel Body |
| SLES (28%) | 70.0 | 70.0 |
| Lauric isopropanolamide | 3.0 | 2.88 |
| Coconut monoethanolamide | 3.0 | 2.88 |
| Titanised mica | – | 0.50 |
| Cationic guar gum | 0.5 | 0.5 |
| Hydroxy propyl guar gum | 0.5 | 0.5 |
| Perfume, dyes, preservatives, etc. | q.s. | q.s. |
| Water to | 100.0% | 100.0% |

## EXAMPLE 6

| Ingredient | % w/w | |
|---|---|---|
| | First Gel Body | Second Gel Body |
| SLES (28%) | 70.0 | 70.0 |
| Lauric isopropanolamide | 3.0 | 2.88 |
| Coconut monoethanolamide | 3.0 | 2.88 |
| Titanised mica | – | 0.50 |
| Cationic guar gum | 0.5 | 0.5 |
| Hydroxy propyl guar gum | 0.5 | 0.5 |
| Piroctone Olamine | – | 2.0 |
| Perfume, dyes, preservatives, etc. | q.s. | q.s. |
| Water to | 100.0% | 100.0 |

## EXAMPLE 7

| Ingredient | % w/w Gel body |
|---|---|
| SLES(28%) | 70.00 |
| Cocamidopropyl betaine(35%) | 1.50 |
| Lauric isopropanolamide | 2.80 |
| Coconut monoethanolamide | 2.80 |
| Cationic guar gum | 0.50 |
| Hydroxy ethyl cellulose | 0.50 |
| Perfume, dyes, preservatives etc | q.s. |
| Water | to 100.00 |

**CONTROL**

| Ingredient | % w/w Gel body |
|---|---|
| SLES (28%) | 70.00 |
| Cocamidopropyl betaine(35%) | 1.50 |
| Lauric isopropanolamide | 2.80 |
| Coconut monoethanolamide | 2.80 |
| Cationic guar gum | 0.50 |
| Perfume, dyes, preservatives etc. | q.s. |
| Water | to 100.00 |

**Test Data**

**Viscosity levels in presence of high and low sulphate levels**

| Composition | Viscocity (cps), $mPa \cdot s$ 25°C | |
|---|---|---|
| | LOW $SO_4^{2-}$ | HIGH $SO_4^{2-}$ |
| Control | 200,000 | 90,000 |
| Example 7 | 250,000 | 235,000 |

Claims

1. A shampoo composition in rigid gel form, comprising from 5 to 25% w/w of anionic detergents (based upon 100% active material) from 0.5 to 7% by weight of the total composition of a thickening agent which has secondary detergent properties, and from 0.1 to 5% by weight of a stabilising humectant polymer selected from non-ionic guar gums, hydroxy $C_{1-6}$ alkyl ethers and hydroxy $C_{1-6}$ alkyl esters of cellulose.

2. A composition according to claim 1 in which the humectant polymer comprises hydroxyl propyl guar gum or hydroxy ethyl cellulose.

3. A composition according to claim 1 or claim 2, containing about 0.5% by weight of humectant polymer.

4. A composition according to anyone of claims 1 to 3, containing an anti-dandruff agent.

5. A composition according to claim 4, which comprises a first gel body containing the detergent and humectant polymer, and a second gel body contiguous with the first gel body, containing the anti-dandruff agent and, optionally, the same humectant polymer as in the first body.

6. A composition according to claim 5 in which the first gel body is transparent or translucent and the second gel body is substantially opaque.

7. A composition according to claim 5 or claim 6 in which the weight ratio of the first gel body to the second gel body is from 1:9 to 9:1.

## Revendications

1. Composition de shampooing sous forme de gel rigide, caractérisée en ce qu'elle comprend de 5 à 25 % en p/p de détergent anionique par rapport à 100 % de matière active, de 0,5 à 7 % en poids de la composition totale d'un agent épaississant qui a des propriétés détergentes secondaires et de 0,1 à 5 % en poids d'un polymère humectant stabilisant choisi parmi des éthers hydroxy-alkylique en $C_{1-6}$ et esters hydroxy-alkylique en $C_{1-6}$ de cellulose et des gommes guar non ioniques.

2. Composition suivant la revendication 1, caractérisée en ce que le polymère humectant comprend une gomme hydroxy-propyl guar ou un hydroxyéthylcellulose.

3. Composition suivant la revendication 1 ou la revendication 2, caractérisée en ce qu'elle contient environ 0,5 % en poids de polymère humectant.

4. Composition suivant l'une quelconque des revendications 1 à 3, caractérisée en ce qu'elle contient un agent anti-pelliculaire.

5. Composition suivant la revendication 4, caractérisée en ce qu'elle comprend un premier corps de gel contenant le détergent et le polymère humectant et un second corps de gel contigu au premier corps de gel, contenant l'agent anti-pelliculaire et éventuellement, le même polymère humectant que dans le premier corps.

6. Composition suivant la revendication 5, caractérisée en ce que le premier corps de gel est transparent ou translucide et que le second corps de gel est pratiquement opaque.

7. Composition suivant la revendication 5 ou la revendication 6, caractérisée en ce que le rapport pondéral du premier corps de gel au second corps de gel est compris entre 1:9 et 9:1.

## Ansprüche

1. Shampoo-Zusammensetzung in Form eines starren Gels, umfassend 5 bis 25 Gew.-% anionisches Detergents (bezogen auf 100 % aktives Material), 0,5 bis 7 Gew.-% der gesamten Zusammensetzung eines Verdickungsmittels, das sekundäre Detergents-Eigenschaften aufweist, und 0,1 bis 5 Gew.-% eines stabilisierenden befeuchtenden Polymerisats, ausgewählt aus nichtionischen Guar-Gummen, Hydroxy-$C_{1-6}$-Alkylethern und Hydroxy-$C_{1-6}$-Alkylestern von Cellulose.

2. Zusammensetzung nach Anspruch 1, in der das befeuchtende Polymerisat Hydroxypropyl-Guar-Gummi oder Hydroxyethyl-cellulose umfaßt.

3. Zusammensetzung nach Anspruch 1 oder Anspruch 2, enthaltend etwa 0,5 Gew.-% des befeuchtenden Polymerisats.

4. Zusammensetzung nach einem der Ansprüche 1 bis 3, enthaltend ein Antischuppenmittel.

5. Zusammensetzung nach Anspruch 4, umfassend einen ersten Gelkörper, der das Detergents und das befeuchtende Polymerisat enthält, und einen zweiten, dem ersten Gelkörper benachbarten Gelkörper, der das Antischuppenmittel und gegebenenfalls das gleiche befeuchtende Polymerisat wie der erste Körper enthält.

6. Zusammensetzung nach Anspruch 5, in der der erste Gelkörper transparent oder durchscheinend und der zweite Gelkörper im wesentlichen trübe ist.

7. Zusammensetzung nach Anspruch 5 oder Anspruch 6, in der das Gewichtsverhältnis des ersten Gelkörpers zum zweiten Gelkörper 1:9 bis 9:1 beträgt.